# EUROPÄISCHE PATENTSCHRIFT

(11) **EP 3 487 978 B1**
(45) Veröffentlichungstag und Bekanntmachung des Hinweises auf die Patenterteilung: **09.09.2020**
(21) Anmeldenummer: 17739917.7
(22) Anmeldetag: 03.07.2017
(51) Int. Cl.: C12M 1/00, C12M 1/36

(54) **DURCH BESTRAHLUNG STERILISIERBARES EINWEG-BIOREAKTORSYSTEM UND VERFAHREN ZUR QUALITÄTSSICHERUNG EINES EINWEG-BIOREAKTORSYSTEMS**
RADIATION-STERILISABLE DISPOSABLE BIOREACTOR SYSTEM AND METHOD FOR QUALITY ASSURANCE OF A DISPOSABLE BIOREACTOR SYSTEM
SYSTÈME DE BIORÉACTEUR À USAGE UNIQUE STÉRILISABLE PAR IRRADIATION ET PROCÉDÉ DE CONTRÔLE DE QUALITÉ D'UN SYSTÈME DE BIORÉACTEUR

(30) Priorität: 20.07.2016 DE 102016113412
(43) Veröffentlichungstag der Anmeldung: 29.05.2019
(73) Patentinhaber: Sartorius Stedim Biotech GmbH, 37079 Göttingen (DE)
(72) Erfinder: GRIMM, Christian, 37308 Heilbad Heiligenstadt (DE); GAO, Wei, 37079 Göttingen (DE); LEUPOLD, Marco, 37079 Göttingen (DE)
(74) Vertreter: Vigand, Philippe
(86) Internationale Anmeldenummer: PCT/EP2017/066519
(87) Internationale Veröffentlichungsnummer: WO 2018/015136

(56) Entgegenhaltungen:
- WO-A1-2017/055164
- US-A1- 2008 024 310
- US-A1- 2009 204 250
- US-A1- 2009 273 447
- US-A1- 2015 137 992
- US-B2- 8 963 684

## Beschreibung

Die Erfindung betrifft ein durch Bestrahlung sterilisierbares Einweg-Bioreaktorsystem, das zur Verwendung in einem biopharmazeutischen Prozess vorgesehen ist. Die Erfindung betrifft ferner ein Verfahren zur Qualitätssicherung eines solchen Einweg-Bioreaktorsystems.

Die Trends der letzten Jahre in der biopharmazeutischen Industrie weisen verstärkt in die Richtung der Verwendung von Einweg-Komponenten (engl.: single use components, Abk.: SU-Komponenten). Diese werden nun auch nicht mehr nur im Bereich der Produkt- und Prozessentwicklung eingesetzt, sondern auch im Bereich der klinischen Prüfmusterherstellung (engl.: Clinical Trial Manufacturing, Abk.: CTM) für das Zulassungsverfahren und sogar in der kommerziellen "Guten Herstellungspraxis" (engl.: Good Manufacturing Practice, Abk.: GMP) bei der Produktion von Arzneimitteln.

Bei der kommerziellen Fertigung, wie beispielsweise bei der Kultivierung von tierischen Zellen in einem Bioreaktor, finden im Wesentlichen zwei verschiedene Ansätze der Nutzung von Einweg-Komponenten Einzug. Zum einen setzen gerade bei neuen Fabriken, Standorten oder Unternehmen speziell in Asien die Betreiber auf einen vollen Single-Use-Ansatz, bei dem die ganze Herstellungskette nahezu ausschließlich mit Einweg-Komponenten abgedeckt wird. Sind zum anderen klassische Geräte aus Edelstahl bereits vorhanden, sollen diese in der Regel aufgrund des hohen Anschaffungspreises auch genutzt werden. In diesem Fall kommt es zum Einsatz von so genannten Hybridanalagen aus bestehenden Edelstahlkomponenten und jeweils neu hinzukommenden Einweg-Komponenten.

Insbesondere im Bereich der klinische Prüfmusterherstellung oder sogar im Umfeld der in den USA gebräuchlichen Methode der Überprüfung der momentanen Qualitätsstandards im Bereich Herstellung (engl.: Current Good Manufacturing Practice, Abk.: cGMP) ist es unerlässlich, dass die Integrität des Reaktionsgefäßes gegeben ist und somit sowohl ein Eindringen von Fremdorganismen als auch eine Austreten des Reaktionsmediums aus dem Reaktor verhindert werden kann. Bei Edelstahlreaktoren wird dies beispielsweise nach Reinigung, Installation und Dampfsterilisation durch Beaufschlagen eines Prüfdruckes und der Ermittlung des Druckabfalls über die Zeit als Messparameter sichergestellt. Diese Druckprüfung wird vom Betreiber der Anlage erfasst und geeignet dokumentiert. Bei Einweg-Bioreaktoren oder anderen aus Kunststoff hergestellten Systemen erfolgt dies direkt vor Benutzung auf ähnliche Weise. Direkt nach Installation des Behälters, wird das System über eine Schlauchverbindung aseptisch an eine Druckprüfungseinrichtung angeschlossen. Es sind Prüfgeräte erhältlich, mit denen sowohl die Druckprüfung als auch eine cGMP-konforme Dokumentation gewährleistet werden können, z.B. der "Sartocheck 4 plus Bag Tester" von Sartorius.

Da die Einweg-Systeme im Gegensatz zu Edelstahl-Systemen keine ortsfesten Anlagen sind, sind die Herstellung und der Transport der Behälter eine ernst zu nehmende Quelle von möglichen Leckagen. Um dem vorzubeugen, wird teilweise schon bei der Produktion von Einweg-Behältern eine Überprüfung der Behälterintegrität durchgeführt. Dazu werden beim Hersteller ähnliche Systeme verwendet, wie sie auch beim späteren Anwender vor der Benutzung zum Einsatz kommen. Wesentlicher Nachteil der bisherigen Methode ist, dass die Informationen zum Integritätstest nicht am Behälter selbst abgelegt sind und deshalb mühsam über die Losnummer (engl.: lot number) rückverfolgt werden müssen.

Ein Einweg-Bioreaktorsystem mit den Merkmalen des Oberbegriffs des Anspruchs 1 ist in der US 2009/0273447 A1 gezeigt. Ein gammastrahlungsbeständiger RFID-Tag, der eine nichtflüchtige Speicherkomponente umfasst, ist an einer für einen biologischen Prozess vorgesehenen Einweg-Komponente angebracht. In den RFID-Tag werden bestimmte Informationen über die Einweg-Komponente eingegeben. Die Einweg-Komponente und der RFID-Tag werden durch Gammastrahlung sterilisiert. Die Informationen werden danach aus dem RFID-Tag ausgelesen und einem Kalibrierungssystem für den biologischen Prozess zugeführt.

Ein Einweg-Bioreaktorsystem mit den Merkmalen des Oberbegriffs des Anspruchs 1 ist auch in der US 2009/0204250 A1 gezeigt. Mehrere RFID-Sensoren sind in Einweg-Komponenten eingebettet. Jeder RFID-Sensor ist konfiguriert, um Multiparameter-Messwerte für mindestens eine der Einweg-Komponenten bereitzustellen. Außerdem stellt jeder RFID-Sensor eine digitale Identifizierung für die Einwegkomponente und ihren jeweiligen RFID-Sensor bereit. Die Multiparameter-Messwerte und die digitale Identifizierung für die Vielzahl von Einwegkomponenten werden von einem RFID-Schreib-/Lesegerät ausgelesen.

In einem anderen Zusammenhang ist aus der US 8 963 684 B2 ein gammasterilisierbares RFID-System bekannt, mit dem nicht vom Originalhersteller hergestellte Einweg-Bioprozess-Komponenten erkannt und daraufhin ein nicht autorisierter Betrieb verhindert werden können. Zu diesem Zweck wird ein FeRAM-Chip (engl.: ferroelectric random access memory chip) benutzt, um fehlerkorrigierbare Informationen auf einem RFID-Tag zu speichern, der an einer Einweg-Bioprozess-Komponente angebracht ist. Die Information bleibt in der Regel auch nach der Gammasterilisation des RFID-Tags und der Einweg-Bioprozess-Komponente im Speicherchip erhalten und kann gegebenenfalls korrigiert werden. Es ist auch ein Verfahren zur Authentifizierung der Einweg-Bioprozess-Komponente beschrieben, welches das Haftungsrisiko des Originalherstellers vermindern soll. Besteht eine Komponente einen Authentifizierungstest nicht, wird eine Warnung an den Benutzer ausgegeben oder der Bioprozess wird angehalten. Auf diese Weise sollen sich minderwertige Fälschungen erkennen lassen und Benutzer in diesem Fall davon abgehalten werden, eine ungerechtfertigte Reklamation beim Originalhersteller geltend zu machen.

Aufgabe der Erfindung ist es, speziell die Dokumentation der Integritätsprüfung eines Einweg-Bioreaktorsystems, die in einem biopharmazeutischen Prozess zum Einsatz kommt, zu verbessern und leichter verfügbar zu machen.

Gelöst wird diese Aufgabe durch ein Einweg-Bioreaktorsystem mit den Merkmalen des Anspruchs 1 sowie durch ein Verfahren zur Qualitätssicherung eines Einweg-Bioreaktorsystems mit den Merkmalen des Anspruchs 12. Vorteilhafte und zweckmäßige Ausgestaltungen des erfindungsgemäßen Einweg-Bioreaktorsystems und des erfindungsgemäßen Verfahrens sind in den zugehörigen Unteransprüchen angegeben.

Das erfindungsgemäße Einweg-Bioreaktorsystem ist zur Verwendung in einem biopharmazeutischen Prozess vorgesehen und durch Bestrahlung sterilisierbar. Das Einweg-Bioreaktorsystem umfasst einen RFID-Tag und einen grundsätzlich wiederbeschreibbaren Speicher, in dem Daten bezüglich einer Integritätsprüfung des Einweg-Bioreaktorsystems abgelegt sind.

Unter einem Einweg-Bioreaktorsystem ist hier nicht zwingend ein vollständiges, voll funktionsfähiges System zu verstehen. Der Begriff "Einweg-Bioreaktorsystem" soll hier stellvertretend für eine oder mehrere wesentliche Einweg-Komponenten stehen, die zusammen für einen Einsatz in einem biopharmazeutischen Prozess vorgesehen sind. Insbesondere soll das Einweg-Bioreaktorsystem einen Behälter (Bag) zur Verwendung in einem Bioreaktor umfassen.

Der Begriff RFID-Tag ist nicht einschränkend zu verstehen und soll alle Typen von Transpondereinheiten, die drahtlos oder kontaktlos betreibbar und/oder auslesbar sind, und alle drahtlosen Übertragungsarten bzw. -standards einschließen, wie etwa NFC (Near Field Communication) etc.

Die Erfindung beruht auf der Erkenntnis, dass es elektronische Speicher gibt, die den Prozess einer Sterilisation eines Einweg-Bioreaktorsystems durch Bestrahlung weitgehend unbeschadet überstehen. Dies eröffnet die Möglichkeit einer dynamisch gestalteten, am Produkt verbleibenden Dokumentation. Das bedeutet, dass nach einem ersten Ablegen von Daten im Speicher die Informationen nach der Bestrahlungssterilisation geändert und/oder ergänzt werden können. So lässt sich die Historie des Einweg-Bioreaktorsystems sorgfältig dokumentieren und für Prüfzwecke leichter verfügbar machen.

Durch die Erfindung wird somit die Prozesssicherheit erhöht, da akkurate Daten über den Zustand bzw. die relevanten Eigenschaften der Komponente(n) des Einweg-Bioreaktorsystems sofort und an Ort und Stelle verfügbar sind. Dies führt einerseits zu weniger Reklamationen beim Hersteller des Einweg-Bioreaktorsystems und andererseits zu einer Verbesserung der Lieferkette.

Der Speicher, in dem die Daten vor und nach einer Bestrahlungssterilisation des Einweg-Bioreaktorsystems abgespeichert werden, ist vorzugsweise Bestandteil des RFID-Tags, sodass kein separates elektronisches Bauteil in das Einweg-Bioreaktorsystem integriert werden muss.

Als Speicher für die vorgesehenen Zwecke eignet sich ein FeRAM-Speicher. Es hat sich gezeigt, dass dieser Speichertyp weitestgehend resistent gegen die bei der Sterilisation von Einweg-Komponenten verwendeten Strahlen(dosen) ist.

In dem Speicher sind vorteilhaft auch produktrelevante Daten abgelegt, insbesondere ein Produkt-Identifikationscode und/oder ein Herstellungsdatum und/oder ein Haltbarkeitsdatum. Diese Daten können sich auf eine oder mehrere Komponenten des Einweg-Bioreaktorsystems beziehen.

In einer vorteilhaften Ausgestaltung des Speichers des erfindungsgemäßen Einweg-Bioreaktorsystems weist dieser einen Schreibschutz auf, der nach seiner Aktivierung ein Löschen und Überschreiben der im Speicher abgelegten Daten verhindert.

Der Speicher ist gemäß der Erfindung in einen freien, beschreibbaren Bereich und einen gesperrten Bereich, der nicht mehr beschreibbar ist, unterteilt. Im gesperrten Bereich können Herstellerdaten und Informationen über die Integritätsprüfung des Einweg-Bioreaktorsystems abgelegt sein, die nicht verändert werden sollen, während in den freien Speicher Informationen und/oder Messdaten, die später vom Kunden z.B. während des bestimmungsgemäßen Gebrauchs des Einweg-Bioreaktorsystems ermittelt werden, geschrieben werden können.

Es hat sich gezeigt, dass bestimmte Batterietypen den Prozess einer Sterilisation des Einweg-Bioreaktorsystems durch Bestrahlung weitgehend unbeschadet überstehen. Basierend auf dieser überraschenden Erkenntnis verfügt der RFID-Tag in einer vorteilhaften Ausgestaltung über eine interne Batterie. Diese Batterie kann die Dokumentation der Historie (Produktion, Integritätsprüfung, Verpackung, Sterilisation, Lagerung, Versand) des Einweg-Bioreaktorsystems unterstützen.

Zur Dokumentation und späteren Auswertungen der Integritätsprüfung und/oder der Lagerbedingungen können vorteilhaft Sensoren zur Erfassung von bestimmten Ereignissen und/oder Umgebungsbedingungen, die Bestandteile des erfindungsgemäßen Einweg-Bioreaktorsystems sind, verwendet werden.

Die Erfindung schafft auch eine Vorrichtungsanordnung mit einer außerhalb des Einweg-Bioreaktorsystems an einer Verpackung angebrachten Akkumulatoreinheit, die auf lösbare Weise elektrisch leitend mit dem RFID-Tag verbunden ist.

Die Akkumulatoreinheit ermöglicht es, die Historie des Einweg-Bioreaktorsystems über einen längeren Zeitraum zu dokumentieren, insbesondere im Falle einer langen Lagerung des Einweg-Bioreaktorsystems. Da die Akkumulatoreinheit wiederaufladbar ist, kann sie auch nach einem etwaigen, durch die Bestrahlungssterilisation des Einweg-Bioreaktorsystems bedingten Entladen zur Stromversorgung des RFID-Tags und etwaiger Sensoren des Einweg-Bioreaktorsystems dienen.

Besonders bevorzugt ist eine Akkumulatoreinheit mit einer Einrichtung zur drahtlosen Aufladung durch eine externe Energiequelle. Die Akkumulatoreinheit kann in diesem Fall im verpackten Zustand des Einweg-Bioreaktorsystems aufgeladen werden, ohne dass eine Kabelverbindung oder dergleichen hergestellt werden muss.

Vorzugsweise ist die Akkumulatoreinheit an einer inneren Seite einer äußeren Verpackung, z.B. in einer Innentasche eines Kartons, angebracht. Die Unterbrechung der lösbaren elektrischen Anbindung der Akkumulatoreinheit kann erkannt und als Zeitpunkt des Auspackens und der Benutzung des Einweg-Bioreaktorsystems dokumentiert werden.

Die erfindungsgemäße Vorrichtungsanordnung kann ergänzt werden durch ein Schreibgerät, das eingerichtet ist zum drahtlosen Schreiben von Daten in den Speicher des Einweg-Bioreaktorsystems sowie ein Lesegerät, das eingerichtet ist zum drahtlosen Auslesen von im Speicher abgelegten Daten. Selbstverständlich können das Schreibgerät und das Lesegerät als kombiniertes Schreib-/Lesegerät ausgebildet sein. Das Auslesen der Daten und deren Einbeziehung in die Prozesssteuerung ermöglicht auch eine Kontrollfunktion vor dem Einsatz des Einweg-Bioreaktorsystems. Anhand der Daten kann sichergestellt werden, dass die verwendeten Komponenten tatsächlich zusammenpassen bzw. kompatibel zueinander sind. Beispielsweise kann nach dem Auslesen der Daten davor gewarnt werden, einen 200-Liter-Beutel in eine Hardware einzulegen, die nur für einen 50-Liter-Beutel ausgelegt ist.

Die Aufgabe der Erfindung wird auch gelöst durch ein Verfahren zur Qualitätssicherung eines erfindungsgemäßen Einweg-Bioreaktorsystems, das zur Verwendung in einem biopharmazeutischen Prozess vorgesehen ist. Das erfindungsgemäße Qualitätssicherungsverfahren umfasst folgende Schritte:
- Versehen des Einweg-Bioreaktorsystems mit einem RFID-Tag und einem grundsätzlich wiederbeschreibbaren Speicher, insbesondere vom Typ FeRAM, der vorzugsweise Bestandteil des RFID-Tags ist;
- Durchführen einer Integritätsprüfung wenigstens einer Komponente des Einweg-Bioreaktorsystems vor einer Sterilisation des Einweg-Bioreaktorsystems durch Bestrahlen; und
- Schreiben von Daten bezüglich der Integritätsprüfung in den Speicher vor der Sterilisation des Einweg-Bioreaktorsystems.

Bezüglich der Vorteile des erfindungsgemäßen Verfahrens wird auf die entsprechenden vorstehenden Ausführungen zum erfindungsgemäßen Einweg-Bioreaktorsystem bzw. zur erfindungsgemäßen Vorrichtungsanordnung verwiesen.

Wie bereits erläutert, können im Rahmen einer Dokumentation der Historie des Einweg-Bioreaktorsystems Informationen über die Durchführung der Integritätsprüfung und/oder über Lagerbedingungen des Einweg-Bioreaktorsystems in den Speicher geschrieben werden.

Ist der Speicher des Einweg-Bioreaktorsystems mit einer entsprechenden Funktionalität ausgestattet, kann vor dem Versand des Einweg-Bioreaktorsystems an einen Kunden ein Schreibschutz des Speichers aktiviert werden. Dadurch bleiben bestimmte Herstellerdaten geschützt.

Gemäß einer Weiterbildung des Erfindungsgedankens kann das Einweg-Bioreaktorsystem mit einem oder mehreren Sensoren bestimmte Ereignisse und/oder Umgebungsbedingungen erfassen und zugehörige Daten in den Speicher schreiben. Dabei wird das Einweg-Bioreaktorsystem über eine externe Akkumulatoreinheit mit Energie versorgt, solange diese genug elektrische Leistung bereitstellen kann.

Gegebenenfalls kann eine interne Batterie des RFID-Tags die Energieversorgung übernehmen, sobald die Energie der Akkumulatoreinheit einen bestimmten Grenzwert unterschreitet.

Wie bereits erläutert soll die Akkumulatoreinheit so eingerichtet sein, dass sie nach der Bestrahlungssterilisation durch eine externe Energiequelle von außen berührungslos erneut aufgeladen werden kann.

Gemäß einer weiteren Fortführung des Erfindungsgedankens soll bei einem Öffnen einer Verpackung, an der die Akkumulatoreinheit angebracht ist, bei einem Entnehmen des Einweg-Bioreaktorsystems aus der Verpackung oder bei einer definierten Erschütterung die elektrische Leitung zwischen der Akkumulatoreinheit und dem Einweg-Bioreaktorsystem unterbrochen und der Zeitpunkt dieses Ereignisses in den Speicher geschrieben werden. Dieser Zeitpunkt soll das Auspacken und die Benutzung dokumentieren, oder dass ab diesem Zeitpunkt die Integrität des Einweg-Bioreaktorsystems seitens des Herstellers nicht mehr gewährleistet wird.

Weitere Merkmale und Vorteile der Erfindung ergeben sich aus der nachfolgenden Beschreibung und aus der beigefügten Zeichnung, auf die Bezug genommen wird. In den Zeichnungen zeigen:
- Figur 1 beispielhaft den Lebenszyklus eines erfindungsgemäßen Einweg-Bioreaktorsystems;
- Figur 2 eine Komponente eines Einweg-Bioreaktorsystems mit einem RFID-Tag, einschließlich einer Detailansicht; und
- Figur 3 die Komponente aus Figur 2 in einem Karton mit einer Einrichtung zur drahtlosen Aufladung eines Akkumulators, einschließlich einer Detailansicht.

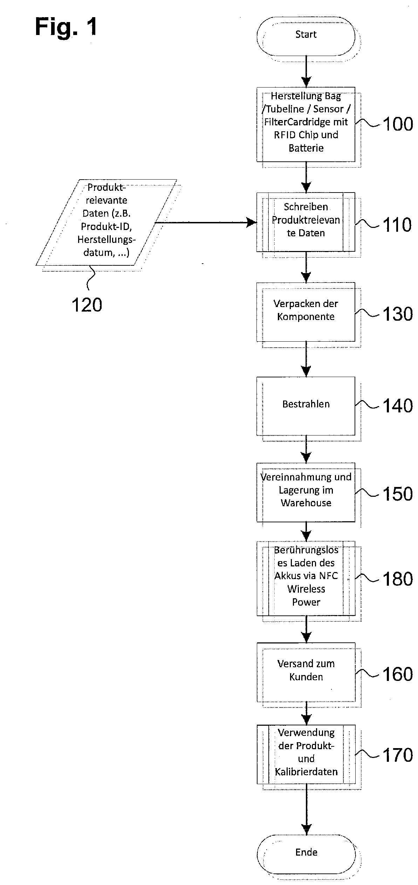

Im Folgenden wird anhand des in Figur 1 gezeigten Flussdiagramms ein typischer Lebenslauf eines Einweg-Bioreaktorsystems beschrieben, beginnend mit dem Schritt 100 der Herstellung des Einweg-Bioreaktorsystems. Das Einweg-Bioreaktorsystem umfasst als Komponenten beispielsweise einen Behälter (Bag) zur Verwendung in einem Bioreaktor und eine Einweg-Sonde. Zumindest der Behälter ist mit einem RFID-Tag (Transponder) versehen, der wiederum über einen grundsätzlich wiederbeschreibbaren Speicher verfügt, insbesondere vom Typ FeRAM.

In einem nächsten Schritt 110 werden mit einem geeigneten Schreibgerät produktrelevante Daten 120 drahtlos in den Speicher des RFID-Tags geschrieben. Die produktrelevanten Daten können einen Produkt-Identifikationscode, das Herstellungsdatum und weitere auf das Einweg-Bioreaktorsystem bezogene Angaben umfassen.

Die Komponenten des Einweg-Bioreaktorsystems mit dem Behälter werden im Schritt 130 verpackt und im Schritt 140 als komplette Einheit, also einschließlich der Verpackung, durch Bestrahlung sterilisiert, insbesondere durch Gammabestrahlung. Ein FeRAM-Speicher übersteht in der Regel eine Sterilisation durch Bestrahlung, d.h. die im Speicher abgelegten Daten sind nach dem Sterilisationsprozess immer noch auslesbar.

Im Schritt 150 wird die sterilisierte Einheit vereinnahmt (bestandmäßige Erfassung) und eingelagert, bevor sie zu gegebener Zeit im Schritt 160 zu einem Kunden versandt wird.

Die einmalige Verwendung des Einweg-Bioreaktorsystems beim Kunden ist durch den Schritt 170 dargestellt.

Der skizzierte Lebenslauf ist ein keiner Weise einschränkend zu verstehen und kann selbstverständlich weitere Schritte aufweisen.

Vor dem Schritt 130 des Verpackens kann seitens des Herstellers die Integrität des Einweg-Bioreaktorsystems überprüft werden, wie unten noch näher erläutert wird. Außerdem können jederzeit die produktrelevanten Daten mit einem geeigneten Lesegerät aus dem Speicher des RFID-Tags ausgelesen und im Rahmen einer Bestands- oder Qualitätsprüfung oder dergleichen ausgewertet werden. Schließlich können bei Bedarf Daten geändert und/oder zusätzliche Daten drahtlos in den Speicher des RFID-Tags geschrieben werden.

Im Folgenden werden die Durchführung, Dokumentation und Speicherung des Tests bzw. der Testergebnisse einer Integritätsprüfung des Einweg-Bioreaktorsystems genauer beschrieben. Zur Überprüfung der Integrität des Einweg-Bioreaktorsystems können im Prinzip folgende Aspekte (einzeln oder in beliebiger Kombination) herangezogen werden: physische Integrität (Dichtheit) des Behälters (Bag); Materialvalidierung inkl. Qualifizierungsparameter (Extractables und Leachables, Bestimmung der Gesamtkeimzahl (Bioburden) etc.); Einhaltung der Umweltbedingungen für Transport und Lagerung (z.B. Grenztemperatur) inkl. Herstelldatum und Haltbarkeitsdatum; Nachverfolgbarkeit von der Produktion über das/die Lager zum Einsatzort; Abgleich, ob Komponenten des Einweg-Bioreaktorsystems zusammenpassen (Bag und Hardware wie Halterung etc.); Parameter in der Dokumentation, z.B. Chargenfreigabe. Die entsprechenden Informationen zur Integrität des Gesamtsystems werden auf dem Speicher des RFID-Tags abgelegt, der in den Behälter integriert und drahtlos betrieben und ausgelesen werden kann.

Nachfolgend wird beispielhaft eine Integritätsprüfung und Dokumentation am Beispiel eines Behälters (Bag) zur Verwendung in einem Bioreaktor beschrieben. Für die Durchführung der Tests wird ein geeignetes Prüfgerät verwendet, wie etwa der eingangs erwähnte "Sartocheck 4 plus Bag Tester", mit dem eine Druckprüfung durchgeführt und eine cGMP-konforme Dokumentation erstellt werden kann. Das Prüfgerät ist mit einer Sende-/Empfangseinheit für RFID-Tags ausgestattet, die in die Gesamtsystemsteuerung des Prüfgeräts bzw. des Prüfprozesses integriert ist.

Die für die Integritätsprüfung vorgesehenen Tests werden in gewohnter Art und Weise mithilfe des Prüfgeräts durchgeführt. Die Testergebnisse, einschließlich des Zeitpunkts der Durchführung und der Rahmenbedingungen der Integritätsprüfung, werden aber nicht nur wie bislang üblich in einer Datenbank abgelegt (in der Regel zusammen mit früheren und zukünftigen Testergebnissen anderer bereits getesteter bzw. noch zu testenden Komponenten). Die Testergebnisse werden darüber hinaus in den Speicher des in den Behälter integrierten RFID-Tags geschrieben. Somit verbleiben die produktrelevanten Daten und die Testergebnisse an einer Einweg-Komponente des Bioreaktorsystems, hier am Behälter, und können bei Bedarf ausgelesen und ergänzt werden.

Für eine Nachverfolgbarkeit des Einweg-Bioreaktorsystems in der Produktion und/oder Lagerung und/oder im Hinblick auf die Überwachung von Umwelteinflüssen und/oder die Einhaltung vorgegebener Lagerbedingungen ist es hilfreich, wenn der RFID-Tag dauerhaft mit einem Energiespeicher verbunden ist.

Die in diesem Zusammenhang naheliegende Maßnahme, in den RFID-Tag selbst einen Energiespeicher zu integrieren, ist wegen der für die Sterilisierung vorgesehenen Gammabestrahlung problematisch. Dem Fachmann ist bekannt, dass übliche Batterien und Akkumulatoren, wie z.B. Lithium-Ionen-Akkumulatoren, durch Gammabestrahlung so stark beschädigt oder beeinträchtigt werden, dass ihre Kapazität signifikant nachlässt.

Grundsätzlich ist es möglich, den Energiespeicher (Batterie, Akkumulator) so groß auszulegen, dass selbst eine drastische Verringerung der Kapazität noch für die vorgesehenen Zwecke ausreicht. Eine weitere Möglichkeit für eine dauerhafte Stromversorgung ist der Einsatz eines (weitgehend) gammastrahlungsresistenten Energiespeichers oder die gezielte Einhaltung eines vorgegebenen Strahlendosisfensters mit Hilfe eines Dosimeters. Die Vorgaben und die tatsächlichen Parameter der Bestrahlung können im Speicher des RFID-Tags abgelegt sein bzw. werden.

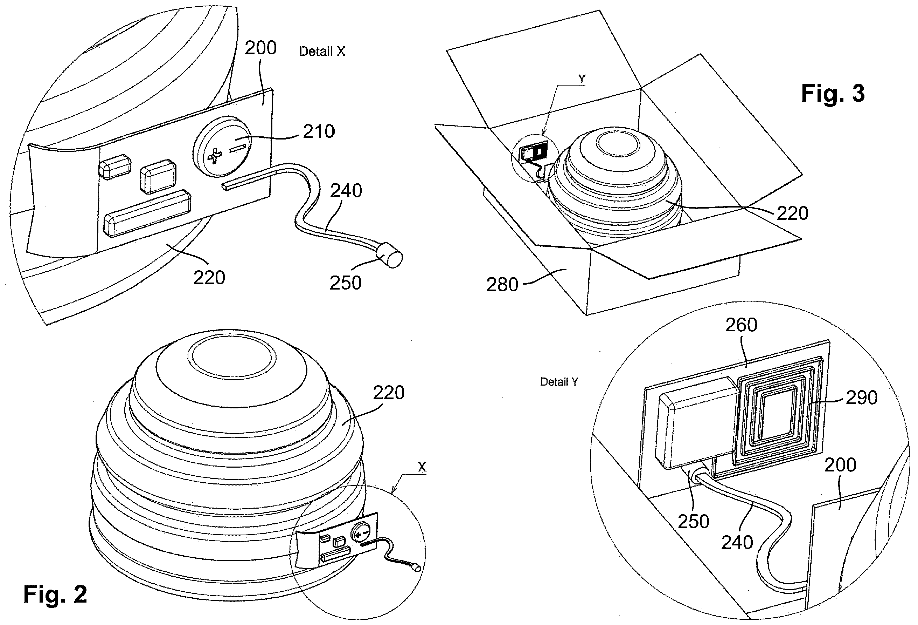

Darüber hinaus kann das Einweg-Bioreaktorsystem durch einen wiederaufladbaren elektrischen Energiespeicher (Akkumulator) ergänzt werden. Ein Beispiel für diese Art der Stromversorgung ist in den Figuren 2 und 3 gezeigt. Ein RFID-Tag 200, der optional mit einer eigenen Batterie 210 ausgestattet sein kann, ist in einen Behälter (Bag) 220 für einen Bioreaktor integriert. Der Behälter 220 befindet sich in einer flexiblen Umverpackung (hier nicht gezeigt). Eine über ein Kabel 240 und eine Steckverbindung 250 oder dergleichen elektrisch leitend mit dem RFID-Tag 200 verbundene Akkumulatoreinheit 260 ist in einer Tasche an der inneren Seite eines steifen Kartons 280 angebracht, in dem der Behälter 220 mit seiner Umverpackung verpackt ist. Die Akkumulatoreinheit 260 verfügt über eine Einrichtung zur drahtlosen Aufladung 290, wie etwa eine geeignete Nahfeldkommunikationsantenne (NFC-Antenne; engl.: Near Field Communication) oder eine andere geeignete Empfangs- oder Kopplungseinheit, vorzugsweise gemäß einem etablierten Standard. Bei der Fertigstellung oder zu einem späteren Zeitpunkt vor der Bestrahlungssterilisation wird die Akkumulatoreinheit 260 erstmals von einer geeigneten externen Energiequelle berührungslos aufgeladen.

Die Verwendung der Akkumulatoreinheit 260 erlaubt es, relevante Ereignisse und Umgebungsbedingungen in der Historie der Einweg-Komponente, hier des Behälters 220, oder des gesamten im Karton 280 verpackten Einweg-Bioreaktorsystems im Speicher des RFID-Tags 200 nicht nur einmalig, sondern über die Zeit, insbesondere in der Zeit vor einer Auslieferung an einen Kunden, zu dokumentieren. Die Erfassung und Speicherung der Daten kann in regelmäßigen oder unregelmäßigen Abständen erfolgen. Die Daten können jederzeit mithilfe eines geeigneten Lesegeräts berührungslos aus dem Speicher des RFID-Tags 200 ausgelesen werden.

Für die Erfassung der Ereignisse und/oder Umgebungsbedingungen können gegebenenfalls Einweg-Sonden verwendet werden, die ohnehin Teil des verpackten Bioreaktorsystems sind und über einen oder mehrere geeignete Sensoren verfügen, wie etwa einen Temperatursensor, einen Feuchtigkeitssensor und/oder einen Strahlungssensor.

Aus den gespeicherten Daten lassen sich später Rückschlüsse auf den aktuellen Zustand ziehen und Aussagen darüber treffen, ob und ggf. wie lange die Komponenten des Einweg-Bioreaktorsystems für den vorgesehenen Einsatzzweck noch tauglich sind.

Wie bereits erwähnt, kann die Akkumulatoreinheit 260 durch die Bestrahlungssterilisation eine Teilschädigung erleiden, was zu einer vollständigen oder teilweisen Entladung führt. Falls die Energie der Akkumulatoreinheit 260 für die vorgesehenen Aufgaben nicht mehr ausreicht, wird dieser Zeitpunkt dokumentiert, d.h. in den Speicher des RFID-Tags geschrieben, und die interne Batterie 210 des RFID-Tags, sofern vorhanden, übernimmt die weitere Stromversorgung.

Dank der Einrichtung 290 zur drahtlosen Aufladung besteht grundsätzlich die Möglichkeit, die Akkumulatoreinheit 260 nach der Bestrahlungssterilisation bei Bedarf durch eine externe Energiequelle von außen berührungslos wieder aufzuladen, wie in Figur 1 durch den Schritt 180 dargestellt. Gegebenenfalls kann auch die Frequenz des Speicherns von Daten und des Auslesens gespeicherter Daten herabgesetzt oder angepasst werden, um die Abdeckung eines gewünschten Mindestzeitraums zu gewährleisten.

Die elektrisch leitende Verbindung zwischen dem RFID-Tag 200 und der Akkumulatoreinheit 260, hier das Kabel 240, ist idealerweise derart gestaltet, dass beim Öffnen des Kartons 280 oder der Umverpackung oder beim Entnehmen des Inhalts der Umverpackung oder bei einer definierten Erschütterung die elektrisch leitende Verbindung unterbrochen wird. Sofern vorhanden, übernimmt dann die interne Batterie 210 des RFID-Tags 200 die weitere Energieversorgung. Der Zeitpunkt der Unterbrechung wird dokumentiert, d.h. in den Speicher des RFID-Tags 200 geschrieben, und kann als Hinweis darauf gedeutet werden, dass das Einweg-Bioreaktorsystem ausgepackt und benutzt wird oder dass die Integrität des Einweg-Bioreaktorsystems nicht mehr gegeben ist.

Vor dem Versand zum Kunden kann im RFID-Tag 200 ein Schreibschutz des Speichers aktiviert werden, um vor Manipulationen oder unbeabsichtigtem Löschen von Daten zu schützen, die im Speicher abgelegt sind. Dadurch ist gewährleistet, dass die korrekten Daten dauerhaft zur Verfügung stehen. Der Kunde kann die Daten bei Bedarf selbst auslesen und entsprechend für seine Zwecke verwenden. Ein anderer Speicherbereich kann dem Kunden aber als (wieder-)beschreibbarer Speicher zur Verfügung gestellt werden, sodass der Kunde eigene Daten im RFID-Tag 200 ablegen und auslesen kann.

## Patentansprüche

1. Durch Bestrahlung sterilisierbares Einweg-Bioreaktorsystem zur Verwendung in einem biopharmazeutischen Prozess, mit einem RFID-Tag und einem grundsätzlich wiederbeschreibbaren Speicher, in dem Daten bezüglich einer Integritätsprüfung des Einweg-Bioreaktorsystems abgelegt sind, **dadurch gekennzeichnet, dass** der Speicher einen freien, beschreibbaren Bereich und einen gesperrten Bereich aufweist, der nicht mehr beschreibbar ist.

2. Einweg-Bioreaktorsystem nach Anspruch 1, **dadurch gekennzeichnet, dass** der Speicher Bestandteil des RFID-Tags ist.

3. Einweg-Bioreaktorsystem nach Anspruch 1 oder 2, **dadurch gekennzeichnet, dass** der Speicher ein FeRAM-Speicher ist.

4. Einweg-Bioreaktorsystem nach einem der vorhergehenden Ansprüche, **dadurch gekennzeichnet, dass** in dem Speicher produktrelevante Daten abgelegt sind, insbesondere ein Produkt-Identifikationscode und/oder ein Herstellungsdatum und/oder ein Haltbarkeitsdatum.

5. Einweg-Bioreaktorsystem nach einem der vorhergehenden Ansprüche, **gekennzeichnet durch** einen Schreibschutz, der nach Aktivierung ein Löschen und Überschreiben der im Speicher abgelegten Daten verhindert.

6. Einweg-Bioreaktorsystem nach einem der vorhergehenden Ansprüche, **dadurch gekennzeichnet, dass** der RFID-Tag über eine interne Batterie verfügt.

7. Einweg-Bioreaktorsystem nach einem der vorhergehenden Ansprüche, mit einem oder mehreren Sensoren zur Erfassung von bestimmten Ereignissen und/oder Umgebungsbedingungen.

8. Vorrichtungsanordnung mit einem Einweg-Bioreaktorsystem nach einem der vorhergehenden Ansprüche sowie einer außerhalb des Einweg-Bioreaktorsystems an einer Verpackung angebrachten Akkumulatoreinheit, die auf lösbare Weise elektrisch leitend mit dem RFID-Tag verbunden ist.

9. Vorrichtungsanordnung nach Anspruch 8, **dadurch gekennzeichnet, dass** die Akkumulatoreinheit eine Einrichtung zur drahtlosen Aufladung durch eine externe Energiequelle aufweist.

10. Vorrichtungsanordnung nach Anspruch 8 oder 9, **dadurch gekennzeichnet, dass** die Akkumulatoreinheit an einer inneren Seite einer äußeren Verpackung angebracht ist.

11. Vorrichtungsanordnung nach einem der Ansprüche 8 bis 10, mit einem Schreibgerät, das eingerichtet ist zum drahtlosen Schreiben von Daten in den Speicher des Einweg-Bioreaktorsystems sowie einem Lesegerät, das eingerichtet ist zum drahtlosen Auslesen von im Speicher abgelegten Daten.

12. Verfahren zur Qualitätssicherung eines Einweg-Bioreaktorsystems nach einem der Ansprüche 1 bis 7, das zur Verwendung in einem biopharmazeutischen Prozess vorgesehen ist, mit folgenden Schritten:
- Versehen des Einweg-Bioreaktorsystems mit einem RFID-Tag und einem grundsätzlich wiederbeschreibbaren Speicher, insbesondere vom Typ FeRAM, der vorzugsweise Bestandteil des RFID-Tags ist;
- Durchführen einer Integritätsprüfung wenigstens einer Komponente des Einweg-Bioreaktorsystems vor einer Sterilisation des Einweg-Bioreaktorsystems durch Bestrahlen; und
- Schreiben von Daten bezüglich der Integritätsprüfung in den Speicher vor der Sterilisation des Einweg-Bioreaktorsystems.

13. Verfahren nach Anspruch 12, **dadurch gekennzeichnet, dass** vor dem Versand des Einweg-Bioreaktorsystems an einen Kunden ein Schreibschutz des Speichers aktiviert wird, und dass vorzugsweise das Einweg-Bioreaktorsystem mit einem oder mehreren Sensoren bestimmte Ereignisse und/oder Umgebungsbedingungen erfasst und zugehörige Daten in den Speicher schreibt, wobei das Einweg-Bioreaktorsystem über eine externe Akkumulatoreinheit mit Energie versorgt wird, und dass weiter vorzugsweise eine interne Batterie des RFID-Tags die Energieversorgung übernimmt, sobald die Energie der Akkumulatoreinheit einen Grenzwert unterschreitet.

14. Verfahren nach Anspruch 13, **dadurch gekennzeichnet, dass** die Akkumulatoreinheit nach der Bestrahlungssterilisation durch eine externe Energiequelle von außen berührungslos erneut aufgeladen wird.

15. Verfahren nach Anspruch 13 oder 14, **dadurch gekennzeichnet, dass** bei einem Öffnen einer Verpackung, an der die Akkumulatoreinheit angebracht ist, bei einem Entnehmen des Einweg-Bioreaktorsystems aus der Verpackung oder bei einer definierten Erschütterung die elektrische Leitung zwischen der Akkumulatoreinheit und dem Einweg-Bioreaktorsystem unterbrochen und der Zeitpunkt dieses Ereignisses in den Speicher geschrieben wird.

## Claims

1. A radiation-sterilizable disposable bioreactor system for use in a biopharmaceutical process, having an RFID tag and a fundamentally rewritable memory, in which data regarding an integrity check of the disposable bioreactor are stored, **characterized in that** the memory has a free, writable region and a blocked region, which is no longer writable.

2. The disposable bioreactor system according to claim 1, **characterized in that** the memory is a component of the RFID tag.

3. The disposable bioreactor system according to claim 1 or 2, **characterized in that** the memory is a FeRAM memory.

4. The disposable bioreactor system according to any of the preceding claims, **characterized in that** product-relevant data are stored in the memory, in particular a product identification code and/or a manufacturing date and/or an expiration date.

5. The disposable bioreactor system according to any of the preceding claims, **characterized by** a write protection which, after activation, prevents deletion and overwriting of the data stored in the memory.

6. The disposable bioreactor system according to any of the preceding claims, **characterized in that** the RFID tag has an internal battery.

7. The disposable bioreactor system according to any of the preceding claims, having one or more sensors for recording specified events and/or environmental conditions.

8. A device assembly having the disposable bioreactor system according to any of the preceding claims and a battery unit mounted on a package outside of the disposable reactor system which is electro-conductively connected to the RFID tag in detachable manner.

9. The device assembly according to claim 8, **characterized in that** the battery unit has a device for wireless charging by means of an external energy source.

10. The device assembly according to claim 8 or 9, **characterized in that** the battery unit is mounted to an exterior package on an inner side.

11. The device assembly according to any of claims 8 to 10, having a writing device that is set up for wireless writing of data to the memory of the disposable bioreactor system and a reading device that is set up for wireless reading of data stored in the memory.

12. A method for quality assurance of a disposable bioreactor system according to any of claims 1 to 7, which is provided for use in a biopharmaceutical process, having the following steps:
- Providing the disposable bioreactor system with an RFID tag and a fundamentally rewritable memory, in particular of the FeRAM type, which is preferably a component of the RFID tag;
- Carrying out an integrity check of at least one component of the disposable bioreactor system prior to a sterilization of the disposable bioreactor system by means of radiation; and
- Writing data regarding the integrity check to the memory prior to the sterilization of the disposable bioreactor system.

13. A method according to claim 12, **characterized in that** prior to the sending of the disposable bioreactor system to a client, a write protection of the memory is activated, and that preferably the disposable bioreactor system records events and/or environmental conditions determined with one or more sensors and writes associated data to the memory, wherein the disposable bioreactor system is supplied with energy by means of an external battery unit, and that further preferably an internal battery of the RFID tag begins supplying energy as soon as the energy of the battery unit falls below a threshold value.

14. The method according to claim 13, **characterized in that** the battery unit is recharged in a non-contact manner from the outside after the radiation sterilization by means of an external energy source.

15. The method according to claim 13 or 14, **characterized in that** in the event of the opening of a package to which the battery unit is mounted, in the event of a removal of the disposable bioreactor system from the package or in the event of a defined jarring, the electrical line between the battery unit and the disposable bioreactor system is disconnected and the time of this event is written to the memory.

## Revendications

1. Système de bioréacteur à usage unique stérilisable par irradiation destiné à être utilisé dans un processus biopharmaceutique, avec une étiquette RFID et une mémoire réinscriptible en principe dans laquelle des données concernant un contrôle d'intégrité du système de bioréacteur à usage unique sont déposées, **caractérisé en ce que** la mémoire présente une zone libre inscriptible et une zone verrouillée qui n'est plus inscriptible.

2. Système de bioréacteur à usage unique selon la revendication 1, **caractérisé en ce que** la mémoire fait partie de l'étiquette RFID.

3. Système de bioréacteur à usage unique selon la revendication 1 ou 2, **caractérisé en ce que** la mémoire est une mémoire FeRAM.

4. Système de bioréacteur à usage unique selon l'une des revendications précédentes, **caractérisé en ce que** des données relatives à un produit sont déposées dans la mémoire, en particulier un code d'identification de produit et/ou une date de fabrication et/ou une date de péremption.

5. Système de bioréacteur à usage unique selon l'une des revendications précédentes, **caractérisé par** une protection en écriture qui après activation empêche une suppression et une réécriture des données déposées dans la mémoire.

6. Système de bioréacteur à usage unique selon l'une des revendications précédentes, **caractérisé en ce que** l'étiquette RFID dispose d'une pile intérieure.

7. Système de bioréacteur à usage unique selon l'une des revendications précédentes, avec un ou plusieurs capteurs pour saisir des événements spécifiques et/ou des conditions ambiantes.

8. Agencement de dispositif avec un système de bioréacteur à usage unique selon l'une des revendications précédentes ainsi qu'avec une unité d'accumulateur appliquée contre un emballage à l'extérieur du système de bioréacteur à usage unique, laquelle unité d'accumulateur est reliée de façon électriquement conductrice et de manière amovible à l'étiquette RFID.

9. Agencement de dispositif selon la revendication 8, **caractérisé en ce que** l'unité d'accumulateur présente un équipement destiné à un rechargement sans fil par une source d'énergie extérieure.

10. Agencement de dispositif selon la revendication 8 ou 9, **caractérisé en ce que** l'unité d'accumulateur est appliquée contre un côté intérieur d'un emballage extérieur.

11. Agencement de dispositif selon l'une des revendications 8 à 10, avec un instrument d'écriture qui est conçu pour l'écriture sans fil de données dans la mémoire du système de bioréacteur à usage unique ainsi qu'avec un instrument de lecture qui est conçu pour la lecture sans fil de données déposées dans la mémoire.

12. Procédé d'assurance qualité d'un système de bioréacteur à usage unique selon l'une des revendications 1 à 7, qui est prévu pour être utilisé dans un processus biopharmaceutique, avec les étapes suivantes consistant à :
- doter le système de bioréacteur à usage unique d'une étiquette RFID et d'une mémoire réinscriptible en principe, en particulier du type FeRAM, qui fait partie de l'étiquette RFID ;
- réaliser un contrôle d'intégrité d'au moins une composante du système de bioréacteur à usage unique avant une stérilisation du système de bioréacteur à usage unique par irradiation ; et
- écrire de données concernant le contrôle d'intégrité dans la mémoire avant la stérilisation du système de bioréacteur à usage unique.

13. Procédé selon la revendication 12, **caractérisé en ce qu'**avant l'envoi du système de bioréacteur à usage unique à un client une protection en écriture de la mémoire est activée, et **en ce que** de préférence le système de bioréacteur à usage unique saisit avec un ou plusieurs capteurs des événements spécifiques et/ou des conditions ambiantes et écrit des données associées dans la mémoire, dans lequel le système de bioréacteur à usage unique est alimenté en énergie via une unité d'accumulateur extérieure, et **en ce qu'**en outre de préférence une pile intérieure de l'étiquette RFID prend en charge l'alimentation en énergie dès lors que l'énergie de l'unité d'accumulateur passe au-dessous d'une valeur limite.

14. Procédé selon la revendication 13, **caractérisé en ce qu'**après la stérilisation par irradiation l'unité d'accumulateur est de nouveau rechargée sans contact depuis l'extérieur par une source d'énergie extérieure.

15. Procédé selon la revendication 13 ou 14, **caractérisé en ce que** lors d'une ouverture d'un emballage contre lequel l'unité d'accumulateur est appliquée, lors d'un retrait du système de bioréacteur à usage unique hors de l'emballage ou lors d'un secousse définie, la conduction électrique entre l'unité d'accumulateur et le système de bioréacteur à usage unique est interrompue et le moment de cet événement est écrit dans la mémoire.
